# EUROPEAN PATENT APPLICATION

(11) **EP 4 394 780 A1**
(43) Date of publication of application: **03.07.2024**
(21) Application number: 22216755.3
(22) Date of filing: 27.12.2022
(51) Int. Cl.: G16C 20/10, G16C 20/20, G16C 20/40, G16C 20/70, G06N 3/0455

(54) **METHODS AND APPARATUSES FOR GENERATING A DIGITAL REPRESENTATION OF CHEMICAL SUBSTANCES, MEASURING PHYSICOCHEMICAL PROPERTIES AND GENERATING CONTROL DATA FOR SYNTHESIZING CHEMICAL SUBSTANCES**

(71) Applicant: BASF SE, 67056 Ludwigshafen am Rhein (DE)
(72) Inventor: Bina, Ali, 40235 Dusseldorf (DE); Niederle, Astrid, Freeport, TX 77541 (US); Smarason, Sigurdur Vidir, 67056 Ludwigshafen am Rhein (DE); Koltzenburg, Sebastian, 67056 Ludwigshafen am Rhein (DE); Kahle, Klaus, 67056 Ludwigshafen am Rhein (DE); Settels, Wolker, 67056 Ludwigshafen am Rhein (DE); Wang, Ning, 13587, Berlin (DE); Gonzales Maldonado, Sandra, 88400, Biberach (DE); Eldred, Rosona, 67056 Ludwigshafen am Rhein (DE)
(74) Representative: Horn Kleimann Waitzhofer Schmid-Dreyer Patent- und Rechtsanwälte PartG mbB

(57) **Abstract**

A method for generating a compressed digital representation of a chemical substance, in particular polymers, includes the steps of receiving (S12) input data being a multimodal representation (2, 12) of a physicochemical property of the chemical substance and indicative of a measurable physicochemical property of the chemical substance; encoding (S13) said input data (2, 12) using a data driven compression model (3, 10) of the chemical substance for generating encoded substance data as a function of the received input data (2, 12); and generating chemical substance data indicative of the measurable physicochemical property of the chemical substance by decoding said encoded substance data using the data driven compression model (3, 10).

## Description

The present disclosure relates to a method for generating a digital representation of a chemical substance. This may involve training aspects of neural networks to represent chemical substances. The present disclosure further relates to a computer program product and to a database search device for identifying chemical substances. Applications of the digital representation involve generating measurement data associated with chemical substances and control data associated with synthesis specifications for chemical substances.

Chemical substances such as polymers come in multiple shapes, sizes and compositions. Small molecules are often represented by their structure (chemical composition). Other basic chemical substances can sometimes be represented by their recipe and a detailed description of their manufacturing process. However, the characteristics of chemical substances such as polymers are often too complex to be represented by their recipe or their structure. It is desirable to provide a more polyvalent manner of representing chemical substances.

In particular, in chemical industries chemical substances such as new polymers are more and more tailored to customer requirements. This requires synthesis of new polymers and subsequently performing measurements on various characteristics. This is very expensive, in addition synthesizing the polymers often generates unnecessary waste, because the success rate for synthesizing a polymer that meets the customer requirements is low. In addition, performing measurements is time consuming and expensive. Thus, there is a need to reduce the number of measurements required to fully analyze a polymer. Digital representations of chemical substances may be a means for reducing efforts in determining or measuring physical or chemical properties of chemical substances, in generating specifications for synthesizing chemical substances and/or in identifying chemical substances for particular purposes.

It is one object of the present invention to provide methods and apparatuses for generating digital representations for chemical substances. Further objects include improved uses and applications of a digital representation in the context of chemical substance synthesis and substance characterization.

The before-mentioned objects are met by the methods and devices according to the independent claims.

According to an aspect, a method for generating a representation of a chemical substance, in particular polymers, is provided, the method including:
receiving input data being a multimodal representation of a physicochemical property of the chemical substance and indicative of a measurable physicochemical property of the chemical substance;
encoding said input data using a data driven compression model of the chemical substance for generating encoded substance data as a function of the received input data; and
generating chemical substance data indicative of the measurable physicochemical property of the chemical substance by decoding said encoded substance data using the data driven compression model.

The method allows to efficiently process data referring to properties of chemical substances. The use of multimodal data in an encoded or compressed form by using the data driven model may improve processing speed and reduce the required amount of resources in terms of energy, material and/or computing power.

Multimodal data relating to a chemical substance includes, for example, various data representing different aspects of the chemical substance. One may contemplate of a first mode or modality being a physical observable, such as a melting temperature, hardness, acidity or the like, and a second mode or modality being a structural aspect, e.g. a chirality of an enantiomer. Spectroscopic aspects may also be regarded as a modality.

In embodiments, the data driven compression model is implemented as a trained neural network. For example, the step of encoding may comprise: providing said input data being a multimodal representation as an input to a trained neural network.

According to an aspect, a method for providing a data driven compression model is disclosed. The method comprises:
providing, as an input to a neural network to be trained, multimodal input data providing a multimodal representation of the chemical substance in a multimodal initial space, the neural network comprising a multimodal variational autoencoder including a multimodal encoder and a multimodal decoder, wherein the multimodal encoder includes multimodal encoder layers having a multimodal encoder weight defining how the multimodal encoder layers transform data, and wherein the multimodal decoder includes multimodal decoder layers having a multimodal decoder weight defining how the multimodal decoder layers transform data;
using the multimodal encoder layers, adjusting a dimensionality of the multimodal input data to obtain multimodal latent data in a latent space;
using the multimodal decoder layers, decoding the multimodal latent data to obtain multimodal reconstructed data in the multimodal initial space;
calculating a loss function for the multimodal variational autoencoder for a current set of multimodal encoder and decoder weights based on the multimodal input data and the multimodal reconstructed data; and
providing the neural network having the current set of multimodal encoder and decoder weights as a data driven compression model.

According to an aspect, a further method for generating a representation of a chemical substance, in particular polymers, in a latent space representation, is provided. The method may involve training a neural network and comprises:
providing, as an input to a neural network to be trained, multimodal input data providing a multimodal representation of the chemical substance in a multimodal initial space, the neural network comprising a multimodal variational autoencoder including a multimodal encoder and a multimodal decoder, wherein the multimodal encoder includes multimodal encoder layers having a multimodal encoder weight defining how the multimodal encoder layers transform data, and wherein the multimodal decoder includes multimodal decoder layers having a multimodal decoder weight defining how the multimodal decoder layers transform data;
using the multimodal encoder layers, adjusting a dimensionality of the multimodal input data to obtain multimodal latent data in a latent space;
using the multimodal decoder layers, decoding the multimodal latent data to obtain multimodal reconstructed data in the multimodal initial space; and
calculating a loss function for the multimodal variational autoencoder for a current set of multimodal encoder and decoder weights based on the multimodal input data and the multimodal reconstructed data.

In embodiments, the method includes the step of outputting configuration data indicative of the trained neural network and/or a latent space representation of the chemical substance as a digital representation of the chemical substance.

The digital representation may further include an identifier of the chemical substance, a plurality of modalities associated with the chemical substance in terms of measurable data for the chemical substance and/pr specification data for synthesizing the chemical substance in terms of recipe data.

Using a multimodal variational autoencoder on multimodal input data allows handling data from multiple modalities at once and bringing the modalities of a same chemical substance together to represent the chemical substance in a single latent space representation. As a result, the neural network is trained to provide a reliable representation of the chemical substance in latent space.

The generated digital representation requires a less amount of data with respect to a complete description including all measurable properties of the chemical substance and thus reduces the amount of required computational and memory resources to process data indictive of the respective chemical substance. Hence, the trained neural network may be considered a data driven compression model.

The combination of the multimodal decoder and encoder may be seen as an embodiment of a data driven compression model, suitable for generating the digital representation of a chemical substance. In particular, an autoencoder as disclosed herein can be regarded an embodiment of a data driven compression model.

The chemical substance can be a form of matter having constant chemical composition and characteristic properties. Examples of chemical substances include polymers and molecules. Polymers can be made up a number of joined-together monomers.

The neural network trained according to the method of the first aspect allows providing a representation of the chemical substance in a latent space representation. The latent space can be an abstract multi-dimensional space which maps what the neural network has learnt from its training data. The latent space representation can be a mathematical representation of the training data with an adjusted (often reduced) dimensionality. "Adjusting" a dimensionality can mean increasing, decreasing, or maintaining the dimensionality the same, in particular to reach a desired (predetermined) dimensionality.

As used herein, a modality is an information relating to the chemical substance from a particular source and/or sensor. The different modalities can be information relating to the chemical substance from different sources and/or sensors. Different modalities can be images of the chemical substance obtained by a camera, spectroscopy images of the chemical substance, recipes of the chemical substance, simulation data of the chemical substance, test data from tests on the chemical substance and the like. Information (data) from the multiple modalities can be expressed as the multimodal input data.

The dimension of the multimodal input is in particular defined through the nature of the measurement. For example, in spectroscopy, the response of the chemical substance is measured in different wavelengths and the range of this wavelength is fixed to an area where it is expected to see a response for chemical substances.

The multimodal initial space can correspond to a space in which the multimodal input data is provided to the neural network. The multimodal initial space can be defined through its dimensionality. The multimodal initial space can be a space in which the data from the different sources and/or sensors is provided directly from the sources and/or sensors or a space in which it has undergone some modifications, such as tuning and/or preprocessing.

The multimodal variational autoencoder (multimodal VAE) can be a variational autoencoder combining and/or taking into account data from different modalities. A structure of the multimodal variational autoencoder can be similar to a structure of a standard variational autoencoder.

The multimodal encoder can be configured to receive the multimodal input data as an input and to adapt the dimensionality thereof to obtain multimodal latent data in the latent space. In particular, the dimensionality of the multimodal latent data is smaller than that of the initial multimodal input data.

The multimodal decoder can be used to decode back the data encoded by the multimodal encoder. In other words, the multimodal decoder receives the multimodal latent data as an input and outputs multimodal reconstructed data in the multimodal initial space (namely, in the same space as the initial multimodal input data).

During the training, a loss function is calculated for the multimodal variational autoencoder. The loss function can be indicative of how well the multimodal variational autoencoder performs. The loss function can correspond to a difference between the multimodal input data and the multimodal reconstructed data. The loss function may be determined through a mixture of expert or through a product of expert technique. In particular, the smaller the loss function, the better the multimodal variational autoencoder.

The current set of multimodal encoder and decoder weights relates to a set of multimodal encoder and decoder weights of a current run (iteration) of the neural network training.

The trained neural network can be used to receive the training data or other types of multimodal data relating to a known chemical substance as an input and to provide a latent space representation thereof as an output.

Applications of having a latent space representation of a chemical substance provided by the trained neural network will be explained further below. Examples include providing a search engine for searching for similarities between chemical substances in the latent space. Other examples include re-engineering of chemical substances and/or the design of new chemical substances.

Further, the latent space representation of a chemical substance can for example be used in an automated production process for producing the chemical substance. Indeed, automated production machines (robots) often require very enriched and compact information about the chemical substance to the produced, which the latent space representation is capable of providing.

According to a further embodiment, the method further includes:
updating the multimodal encoder weight and/or the multimodal decoder weight based on the loss function.

The result of the multimodal variational autoencoder, namely the multimodal latent data and the multimodal reconstructed data, can be varied by modifying the multimodal encoder weight and the multimodal decoder weight.

Updating the multimodal encoder weight and/or the multimodal decoder weight based on the loss function hence allows modifying the result (output) of the multimodal variational autoencoder, in order to reduce the loss function and/or improve the neural network during its training, in particular to provide a latent space representation of input data relating to a chemical substance that is as accurate and/or convenient as possible.

According to a further embodiment, the method further includes:
repeating the steps of providing multimodal input data, adjusting the dimensionality, decoding the multimodal latent data, calculating a loss function, and/or updating the weights to reduce the loss function.

By repeating these steps, the loss function can be reduced more and more until a satisfactory and reliable neural network is obtained. The training of the neural network may end once the loss function is below a predetermined threshold, after a predetermined number of repetitions (runs) of the steps of providing multimodal input data, adjusting the dimensionality, decoding the multimodal latent data, calculating a loss function, and/or updating the weights to reduce the loss function, or the like.

According to a further embodiment, the neural network further includes an individual variational autoencoder assigned to each modality, the individual variational autoencoder including an individual encoder and an individual decoder, wherein the individual encoder includes individual encoder layers having an individual encoder weight defining how the individual encoder layers transform data, and wherein the individual decoder includes individual decoder layers having an individual decoder weight defining how the individual decoder layers transform data, the method further comprising:
training each individual variational autoencoder by:
   inputting into the individual variational autoencoder, individual input data representative only of the assigned modality in an individual initial space;
   using the individual encoder layer, adjusting a dimensionality of the individual input data to obtain individual latent data with a predetermined dimensionality;
   using the individual decoder layer, decoding the individual latent data to obtain individual reconstructed data in the individual initial space;
   comparing the individual input data with the individual reconstructed data to obtain a comparison result;
   updating the individual encoder weight and/or the individual decoder weight based on the comparison result; and
   repeating the steps of inputting individual input data, adjusting a dimensionality of the individual input data, decoding the individual latent data, comparing the data and updating the individual encoder weight and/or the individual decoder weight to reduce the comparison result;
and the method further including:
   using the individual latent data from the multiple individual variational autoencoders as the multimodal input data of the multimodal variational autoencoder.

The individual variational autoencoders can be used to preprocess the data from the different modalities before inputting it into the multimodal variational autoencoder. For example, the individual variational autoencoders perform a preprocessing or tuning of the individual data representing the individual modalities separately. The individual variational autoencoders may bring the data from all the modalities into a same format and/or into a same dimensionality (corresponding to the "predetermined dimensionality") in the individual latent data. This can facilitate a processing by the multimodal variational autoencoder receiving, as the multimodal input data, the individual latent data and/or information on the individual variational autoencoders, such as hyperparameters determined during training of the individual variational autoencoders.

Each individual variational autoencoder (individual VAE) can be a variational autoencoder considering only data from a single modality (the assigned modality). As such, data from each modality is treated separately, by a single individual variational autoencoder. A structure of the individual variational autoencoder can be similar to a structure of a standard variational autoencoder.

The individual encoder can be configured to receive the individual input data of the assigned modality as an input and to adjust (increase, decrease or maintain) the dimensionality thereof to obtain individual latent data in the individual latent space.

The individual decoder can be used to decode back the data encoded by the individual encoder. In other words, the individual decoder receives the individual latent data as an input and outputs individual reconstructed data in the individual initial space (namely, in the same space as the initial individual input data).

During the training, an individual loss function is calculated for the individual variational autoencoder. This individual loss function can be indicative of how well the individual variational autoencoder performs. The individual loss function can correspond to a difference between the individual input data and the individual reconstructed data and be expressed as the comparison result. In particular, the smaller the comparison result, the better the individual variational autoencoder.

Updating the individual encoder weight and/or the individual decoder weight based on the loss function hence allows modifying the result (output) of the individual variational autoencoder, in order to reduce the loss function and/or improve the neural network in training.

According to a further embodiment, the method further includes:
repeating the training of each individual variational autoencoder for a different predetermined dimensionality of the individual latent data; and
selecting the predetermined dimensionality for which the loss function is smallest as the predetermined dimensionality of the final trained neural network.

In particular, the loss function of the multimodal variational autoencoder is calculated for different sets of multimodal input data, which correspond to individual latent data obtained from the individual variational autoencoders for different predetermined dimensionalities. By varying the predetermined dimensionality, the loss function can be reduced. The final trained neural network is the trained neural network. Preferably, the final trained neural network has hyperparameters and/or weights minimizing the loss function. The predetermined dimensionality can be considered as a hyperparameter.

According to a further embodiment, the loss function for the multimodal variational encoder is determined through a mixture of expert or through a product of expert technique.

The mixture of expert technique involves decomposing predictive modeling tasks into sub-tasks, training an expert model on each, developing a gating model that learns which expert to trust based on the input to be predicted, and combines the predictions. The product of expert technique models a probability distribution by combining the output from several simpler distributions.

According to a further embodiment, one of the modalities describing the chemical substance provides: a spectroscopic representation, a rheologic representation, a thermal representation, a chemical representation, a structural representation, a representation of the solubility, a representation of the dispersion, a representation of the viscosity and/or a representation of the surface tension of the chemical substance.

In particular, one modality could correspond to any analysis and/or characterization method of a chemical substance. These include: (i) all type of spectroscopy data such as Fourier Transform infrared spectroscopy (FTIR), Raman spectroscopy, Raman microscopy, ultraviolet spectroscopy, nuclear magnetic resonance spectroscopy (NMR), mass spectroscopy (MS), gas chromatograph - mass spectrometry (GCMS) or the like; (ii) thermal analysis data such as dynamic mechanical analysis (DMTA), differential scanning calorimetry (DSC), thermogravimetric analysis (TGA), dynamic mechanical analysis (DMA) or the like; (iii) structural characterization data, such as X-ray diffraction; and (iv) data from unique analytic techniques measuring for the physical properties of polymers, such as, viscosimeter, surface tension and the like.

FTIR for example always includes errors coming from domain specific sources. The neural network is thus trained such as to be capable of dealing with such errors and of dealing with chemistry data in a more general way.

According to a further embodiment, at least two modalities provided in the multimodal representation of the individual or multimodal input data include data in different formats.

Data in different formats can include image, table, text, and the like. Data in different formats can also include data having different dimensions and/or data being structured or presented differently.

According to a further embodiment, at least part of the multimodal and/or individual input data is sequential data.

In order to deal with sequential data, the neural network may be a convolutional neural network.

According to a further embodiment, the individual input data and/or the multimodal input data includes augmented data.

Augmented data can designate data generated artificially, for example by generating slightly modified copies of initial data. This allows increasing the amount of training data (individual and/or multimodal input data) and hence improving the training of the neural network.

According to a further embodiment, the step of using the individual encoder layers to adjust the dimensionality of the individual input data includes increasing the dimensionality of the individual input data for at least one modality and includes reducing the dimensionality of the individual output data for at least another modality.

Depending on the modality to which they are assigned, the individual encoder layers may either increase or reduce the dimensionality of the individual input data to obtain the individual data at the predetermined dimensionality. For example, data representing some modalities may be a scalar (optionally with an error bar) and the dimensionality thereof is increased to achieve the predetermined dimensionality.

According to a further aspect a method for measuring a physicochemical property of a chemical substance, in particular a desired, preset and/or predetermined physicochemical property of the chemical substance, comprises the steps of:
receiving sensor data indicative of a first measurable physicochemical property of the chemical substance;
encoding said sensor data using a data driven compression model of the chemical substance for generating encoded sensor data; and
generating measurement data indicative of a second measurable physicochemical property of the chemical substance by decoding said encoded sensor data using the data driven compression model.

Preferably, the steps of encoding and decoding are carried out in accordance with the respective steps of the first aspect of this disclosure or its embodiments.

In embodiments, the data driven compression model is implemented to map input data to encoded output data according to the method of the first aspect for generating a representation of a chemical substance, wherein said input data is a multimodal representation of a physicochemical property of the chemical substance, and said encoded output data is a latent space representation of the input data.

Determining properties of chemical substances in the digital latent space representation may avoid measurements and experiments that need to be carried out in otherwise with samples of the respective substance. Further, generated data indicative of physicochemical properties using the presented data driven methods and compression models may be used as training data for artificial intelligence purposes. One aspect of this disclosure is thus also the use of the generated measurement data as training data. While the sensor data may be obtained by a hardware measurement the generated measurement data may be considered synthetic measurement data.

In embodiments, the provided or received sensor data relate to a first type of physical measurements, and the generated measurement data relate to a second type of physical measurements. For example, one my contemplate of providing X-ray diffraction data as received sensor data and near infrared spectra as generated measurement data. The digital representation allows to partly dispense with energy and resource demanding technical processes to measure samples of substances.

Yet according to a further aspect a measurement apparatus for measuring a physicochemical property of a chemical substance, in particular a desired, preset and/or predetermined physicochemical property of the chemical substance, comprises:
an interface device implemented to receive sensor data indicative of a first measurable physicochemical property of the chemical substance;
an encoder device implemented to encode received sensor data and to generate and output encoded sensor data; and
a decoder device implemented to generate measurement data indicative of a second measurable physicochemical property of the chemical substance, and to decode said encoded sensor data.

The encoder device is preferably implemented to map input data to encoded output data according to the method of the first aspect for generating a representation of a chemical substance, wherein said input data is a multimodal representation of a physicochemical property of the chemical substance in the multimodal initial space, and said encoded output data is a latent space representation of the input data.

The decoder device is preferably implemented to map input data to decoded output data according to the method of the first aspect for generating a representation of a chemical substance, wherein said input data is a multimodal latent space representation of a physicochemical property of the chemical substance, and said decoded output data is multimodal reconstructed data in the multimodal initial space.

According to an aspect, a computer program product comprising instructions which, when the program is executed by a computer, cause the computer to carry out the method of the first aspect or according to an embodiment thereof is provided.

A computer program product, such as a computer program means, may be embodied as a memory card, USB stick, CD-ROM, DVD or as a file which may be downloaded from a server in a network. For example, such a file may be provided by transferring the file comprising the computer program product from a wireless communication network.

The embodiments and features described with reference to the training method of the first aspect or an embodiment thereof apply mutatis mutandis to the computer program product of the second aspect.

According to an aspect, a database search device, in particular implemented to identify a chemical substance having a predetermined physicochemical property is provided. The database search device comprises:
a storage unit for storing a database and a trained neural network, the database comprising representations of multiple chemical substances, in particular in a latent space, obtained using the trained neural network. and the trained neural network comprising a encoder and a decoder;
an input unit for receiving search input data indicative of a chemical substance to be searched;
a processor configured to:
   use the encoder to adjust a dimensionality of the search input data to obtain encoded search data in the encoded space;
   compare the encoded search data with the representations of the multiple chemical substances in the database;
   select at least one chemical substance from the multiple chemical substances represented in the database based on a result of the comparison between the encoded search data and the representations of the multiple chemical substances; and
an output unit for outputting an identifier of the selected chemical substance.

The trained neural network may comprise a variational autoencoder including a multimodal encoder and a multimodal decoder.

The database search device can be part of a computer, in particular of a personal computer or of an industrial computer. The trained neural network can be used to provide a latent space representation of chemical substances. In particular, the database includes latent space representations of multiple chemical substances obtained using the trained neural network. For example, the database can be regularly and/or constantly updated as new data regarding chemical substances is obtained.

The storage unit storing the database and the trained neural network may be any type of temporal or permanent storage (memory). The processor may be a central processing unit (CPU) or the like which is configured to access the database and/or execute the neural network stored therein. The input unit can include a user interface to receive the search input data from the user, or it can be a unit that can access the search input data stored in the storage unit or the like.

The search input data is data that has not yet been input into the neural network and/or for which no latent representation has been stored. The search input data may be of the same format as the multimodal input data previously described. The search input data may also be incomplete data representing a chemical substance, which for example only includes the representation of some of the modalities of the chemical substance.

The neural network can be used to bring the search input data in the same latent space representation as the data in the database. This is performed using the multimodal encoder of the neural network, which is in particular capable of combining the multiple modalities of the search input data.

Preferably, the latent search data is in the same representation (and dimensionality) as the data in the database. A comparison of the latent search data with the data in the database (i.e. with the representations of the multiple chemical substances) can be performed by directly comparing the latent search data with the data in the database. For example, the numerical value of the latent search data assigned with each of its dimensionalities can be directly compared with the numerical value of each data in the database assigned with the same dimensionality. The comparison allows determining a resemblance between the latent search data and each representation of the multiple chemical substances in the latent space. A comparison score proportional to the resemblance may be assigned to each representation of the multiple chemical substances in the latent space.

The at least one selected chemical substance can be the chemical substance whose latent space representation in the database is closest (most similar, for example with the highest comparison score) to the latent search data. Multiple selected chemical substances can be the N chemical substances represented in the database that are closest to the latent search data. This relies on the fact that similar chemical substances will have similar latent space representations.

The output unit can be a user interface, such as a display, touchscreen, or the like. The identifier of the selected chemical substance can include a name, chemical composition, reference number or other identification information of the selected chemical substance. The output unit can output the identifier by outputting (displaying) it to a user, storing it in the storage unit or the like.

The database search device can be used to identify chemical substances based on their multimodal representation (search input data) by performing a comparison in latent space.

The database search device can be used to perform re-engineering, namely to find a representation of a chemical substance without knowing its recipe. In this case, once the unknown chemical substance search has been searched, its recipe can be derived from the recipe of the selected chemical substances.

Further, the latent space representation provided by the neural network can reduce the few shot learning problem (the problem of making predictions based on a limited number of samples) by reducing the dimensionality of the input data and by providing an enriched feature space that has been trained on a very big dataset.

According to an embodiment, the neural network is trained according to the method of the first aspect or any embodiment thereof.

The database search device can further be configured to perform the training of the neural network according to the method of the first aspect or any embodiment thereof.

A further aspect of this disclosure involves a method for generating control data indicative of a synthesis specification for a chemical substance, in particular a polymer, comprises:
providing a first synthesis specification for a reference chemical substance;
encoding the first synthesis specification using a data driven compression model into a digital representation of the reference chemical substance;
providing a database comprising a plurality of historical digital representations of historical chemical substances;
determine a similarity score for the historical digital representations with respect to the digital representation of the reference chemical substance;
based on the similarity score, selecting at least one historical representation, and decoding a synthesis specification associated with the least one selected historical representation; and
generating control data indicative of the generated synthesis specification.

A synthesis specification for a chemical substance preferably includes all process and recipe data needed to produce the respective chemical substance. Synthesis specification data my include control data for operating a chemical plant in a machine-readable form.

As a result of the foregoing aspect, a synthesis specification is obtained that may result in a chemical substance similar to the reference chemical substance if the control data is deployed in a chemical plant, i.e. a system for producing a chemical substance according to the control data. The method also provides for an alternative synthesis specification for the reference substance.

In embodiments, the data driven compression model is implemented to map input data to encoded output data according to the method of the first aspect for generating a representation of a chemical substance,, wherein said input data is a multimodal representation of a chemical substance, and said encoded output data is a latent space representation of the input data.

The data base may be comprised in the data base search device according to the foregoing aspect.

In embodiments, the search input data indicates a component of the chemical substance, which is to be replaced by an alternative component, and the selected chemical substance included the alternative component instead of the component to be replaced.

In embodiments, the search input data indicates a qualitative property of the chemical substance. A quality may refer to a classification according to predetermined regulations, e,g. the German Gefahrstoffverordnung - GefStoffV.

In particular, an identifier indicative of the component to be replaced and/or the qualitative property may be a modality in terms of the encoder and decoder of the neural network.

One more aspect relates to a method for generating control data indicative of a synthesis specification for a chemical substance, in particular a polymer, the method comprising:
receiving sensor data indicative of a measurable physicochemical property of the chemical substance;
encoding said sensor data using a data driven compression model of the chemical substance for generating encoded sensor data; and
generating control data indicative of a synthesis specification for the chemical substance by decoding said encoded sensor data using the data driven compression model;
wherein the data driven compression model is implemented to map input data to encoded output data according to the method of the first aspect for generating a representation of a chemical substance, wherein said input data is a multimodal representation of a physicochemical property of the chemical substance, and said encoded output data is, in particular, a latent space representation of the input data.

The presented aspects allow to generate control data for synthesizing a chemical substance according to the desired physicochemical properties without real-word experiments or test runs of chemical plants. The digital representation in terms of a latent space representation thus facilitates the manufacture of plants and operating such for producing chemicals.

In embodiments, the methods for generating control data may include at least one of the steps of:
applying constraints indicative for a process or substance requirement, in particular a requirement of biodegradability for the chemical substance and/or ingredients to produce the chemical substance, of being based on biomass, the exclusion of toxic ingredients and the like; and
validating if the generated synthesis specification meets the constraints, in particular, prior to generating the control data.

The embodiments and features described with reference to the training method aspects of the first aspect or an embodiment thereof apply mutatis mutandis to the database device of the third aspect and the other aspects of methods for determining physicochemical properties, generating control data and/or measurement data.

According to some aspects, the processor is further capable of using the multimodal decoder to decode the latent representation of the at least one selected chemical substance to obtain reconstructed representation data in the multimodal initial space; and the output unit is configured to output the reconstructed representation data. Thereby, a representation of the selected chemical substance is provided in the initial space, which is understandable and analyzable, for example by a user.

The disclosed aspects, in particular allow to replace known polymers with similar performing molecules including the generation of suitable synthesis specifications for the replacement polymer/molecule.

Further possible implementations or alternative solutions of the invention also encompass combinations - that are not explicitly mentioned herein - of features described above or below with regard to the embodiments. The person skilled in the art may also add individual or isolated aspects and features to the most basic form of the invention.

Further embodiments, features and advantages of the present invention will become apparent from the subsequent description and dependent claims, taken in conjunction with the accompanying drawings, in which:
- Fig. 1: shows a first example of a neural network;
- Fig. 2: shows a first embodiment of a method for training the neural network of Fig. 1;
- Fig. 3: shows a second embodiment of a method for training the neural network of Fig. 1;
- Fig. 4: shows a second example of a neural network;
- Fig. 5: shows a first embodiment of a method for training the neural network of Fig. 4;
- Fig. 6: shows a different representation of the training method of Fig. 5;
- Fig. 7: shows a database search device;
- Fig. 8: shows a method of operating the database search device of Fig. 7; and
- Fig. 9: shows an embodiment of a system for producing a chemical substance.

In the Figures, like reference numerals designate like or functionally equivalent elements, unless otherwise indicated.

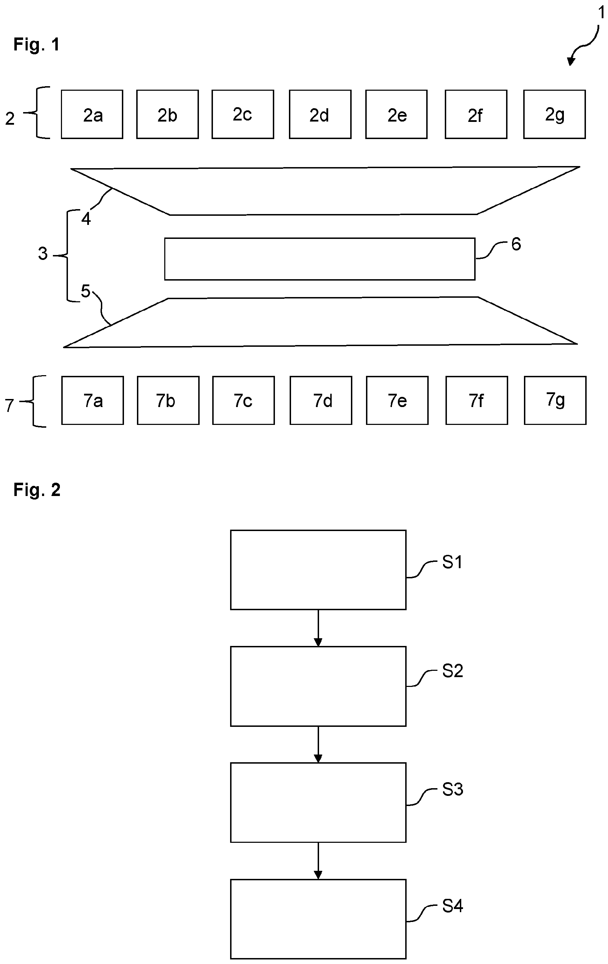

Fig. 1 shows an example of a neural network 1 comprising a multimodal variational autoencoder 3 with a multimodal encoder 4 and a multimodal decoder 5. The neural network 1 is trained according to the method of Fig. 2 so that Fig. 1 and 2 will be described jointly in the following.

It is understood that regarding the following embodiments, the presented neural networks embody a framework for the digital representation of a chemical substance. The deployed artificial neural network may be characterized in terms of its parameters such as numbers of and characteristics of implemented neurons, weights, nodes, connections, and other configurational parameters. The expression "latent space representation" in the context of this application refers to a digital representation of a chemical substance such as a polymer in the following. "Modalities" describing the chemical substance refer to physicochemical properties of the chemical substance that are observable through measurements and can be represented in a digital or computer processable fashion, e.g. being indicative of a spectroscopic representation, a rheologic representation, a thermal representation, a chemical representation, a structural representation, a representation of the solubility, a representation of the dispersion, a representation of the viscosity and/or a representation of the surface tension of the chemical substance.

The multimodal encoder 4 and the multimodal decoder 5 form the interfaces to the latent space representation 6, and are thus a computer-implemented embodiment of a data driven compression model.

In order to train the neural network 1, the neural network 1 receives, as an input, multimodal input data 2 (step S1 of Fig. 2). In the example of Fig. 1, the multimodal input data 2 comprises data representing seven modalities 2a - 2g of a same chemical substance, here a polymer. Modality 2a includes spectroscopy data from spectroscopy measurement, modality 2b includes rheology data, modality 2c includes X-ray diffraction data, modality 2d includes solubility data, modality 2e includes dispersion clay data (which is indicative of the interaction of the chemical substance with a layered structure of clay), modality 2f includes surface tension data, and modality 2g includes viscosity data of the polymer. The data from all dimensionalities was obtained by performing a corresponding measurement on the polymer using sensors. The neural network 1 receives multimodal input data 2 referring to multiple polymers.

The multimodal input data 2 is provided in an initial space. In the initial space, the data from each modality 2a - 2g has its own dimensionality, which here corresponds to the dimensionality of the data as sensed by the sensors. As such, the modalities 2a to 2c have a higher dimensionality (between 5 and 100) than the modalities 2d to 2g (which have only one dimension), which are scalars. Alternatively, in the initial space, the data from each modality 2a - 2g has the same dimensionality (for example, 50).

In a step S2 of Fig. 2, the dimensionality of the multimodal input data 2 is modified using the multimodal encoder 4. The multimodal encoder 4 includes multiple multimodal encoder layers each having a multimodal encoder weight defining a mathematical operation according to which the multimodal encoder 4 transforms the multimodal input data 2. The multimodal encoder weights are some of the parameters that are modified and optimized during the training of the neural network 1, as will be explained further below.

In step S2, the multimodal encoder 4 reduces the number of dimensions of the multimodal input data 2 to obtain multimodal latent data in a latent space 6. The latent space representation of the multimodal input data 2, i.e. the multimodal latent data, comprises 16 dimensions in the present example.

In step S2, the multimodal encoder 4 combines the data from all modalities 2a - 2g to form a single set of data describing the polymer in the latent space 6.

In step S3, the multimodal decoder 5 is used to decode the multimodal latent data to obtain multimodal reconstructed data 7, 7a - 7g in the initial space. This includes modifying the dimensionality of the multimodal latent data to go back to the dimensionality or dimensionalities of the initial multimodal input data 2. The multimodal decoder 5 includes multiple multimodal decoder layers each having a multimodal decoder weight defining a mathematical operation according to which the multimodal decoder 5 the multimodal latent data. The multimodal decoder weights are some of the parameters that are modified and optimized during the training of the neural network 1, as will be explained further below.

In a step S4 of the training method of Fig. 2, a loss function of the multimodal variational autoencoder 3 is calculated. In its simplest form, the loss function indicates a similarity degree between the multimodal input data 2 and the multimodal reconstructed data 7. Alternative manners of calculating the loss function of the multimodal variational autoencoder 3 include a mixture of expert, a mixture of Gaussian and/or a product of expert technique.

The calculated loss function is indicative of how well the neural network 1 is performing during the current run (iteration). The smaller the loss function, the better the neural network 1.

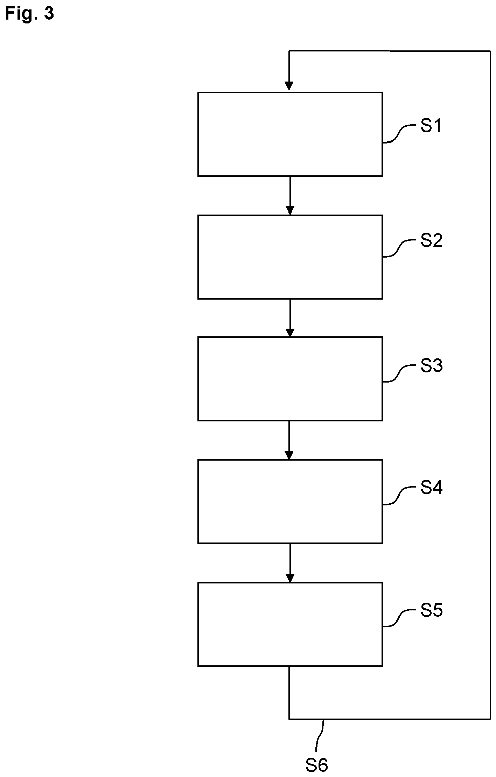

Fig. 3 shows a further embodiment of the method for training the neural network 1 of Fig. 1. The method steps S1 - S4 of Fig. 3 are identical with those of Fig. 2. Depending on the calculated loss function, the neural network may update all or some of the multimodal encoder weights and/or all or some of the multimodal decoder weights in an optional step S5 of Fig. 3. The multimodal encoder weights and/or the multimodal decoder weights are updated through back-propagation.

As shown in Fig. 3, in a step S6, all method steps S1 - S5 can be repeated to reduce the loss function and hence improve the neural network 1. Steps S1 - S5 may be repeated for a predetermined number of runs or until the calculated loss function is smaller than a predetermined loss function threshold. When the training stops, the multimodal encoder weights and the multimodal decoder weights of the run providing the lowest loss function are kept as the weights leading to the best neural network 1. The trained neural network 1 corresponds to this best run and has its multimodal encoder weights and decoder weights.

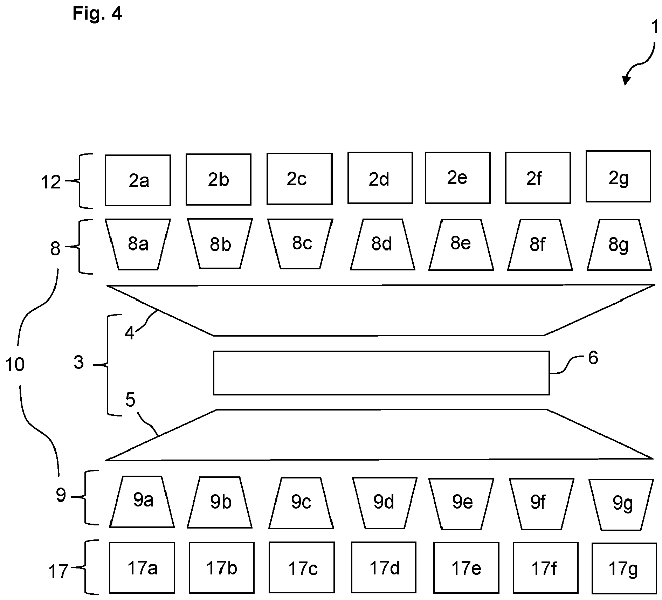

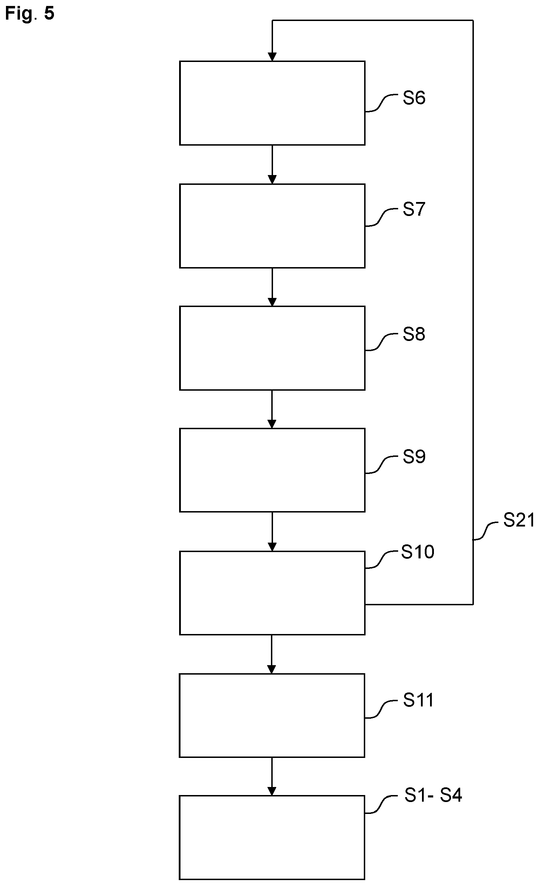

Fig. 4 shows a second example of a neural network 1. Fig. 5 shows an embodiment of a method for training the neural network 1 of Fig. 4. Many elements of the neural network 1 of Fig. 4 and of the method of Fig. 5 are identical with the neural network 1 and the training methods of Fig. 1 to 3 and equally apply for the description of Fig. 4 and 5.

A difference to the neural network 1 of Fig. 1 is that the neural network of Fig. 4 further comprising seven individual variational autoencoders 10 each including an individual encoder 8 and an individual decoder 9. In detail, the individual encoders 8a - 8g and the individual decoders 9a - 9g respectively correspond to the modalities 2a - 2g. The modalities 2a - 2g correspond to the modalities 2a - 2g previously described, but their characterizing data forms individual input data 12 instead of multimodal input data 2. The difference between the individual input data 12 and the multimodal input data 2 is that the individual input data 12 is input into the individual variational autoencoder 10 while the multimodal input data 2 is input into the multimodal variational autoencoder 3. Further, the individual input data 12 includes data of different dimensions for the different modalities, while the multimodal input data 2 may include data of the same dimension for all modalities 2a - 2g.

The individual variational autoencoders 10 are for tuning the data 12 before inputting it into the multimodal variational autoencoder 3. The individual encoders 8a - 8g bring the input data 12 from each modality 2a - 2g into a same predetermined dimensionality, which can be the dimensionality of the latent space 6 (for example, dimensionality 16).

In detail, in a step S6 of Fig. 5, the individual input data 12 representative of each single modality 2a - 2g is input into the corresponding individual encoder 8. This means that the individual input data 12 representative of the modality 2a is input into the corresponding individual encoder 8a, the individual input data 12 representative of the modality 2b is input into the corresponding individual encoder 8b, and so on.

In a step S7 of Fig. 5, each individual encoder 8 modifies the dimensionality of the received individual input data 12 to obtain data with a predetermined dimensionality (for example, 16). The obtained data with the predetermined dimensionality is called "individual latent data" and can correspond to the multimodal input data 2 described in view of Fig. 1.

In a step S8, the individual decoders 9a - 9g are used to reconstruct the individual latent data to obtain individual reconstructed data 17 in the individual initial space (i.e. in the same space as the individual input data 12). The individual reconstructed data 17 includes individual data 17a - 17g for each modality 2a - 2g. The individual reconstructed data 17 can be in the same space as the multimodal reconstructed data 7 of Fig. 1 and be identical thereto, or it can be in a different space (the individual latent space).

In a step S9, the individual input data 12 from each modality 2a - 2g is compared with the corresponding individual reconstructed data 17a - 17g to obtain a comparison result. The better each individual variational autoencoder 10, the more similar its input data 12 and reconstructed data 17. The comparison result may be a loss function.

Accordingly, in a step S10 of Fig. 5, the weights of the individual variational autoencoders 10 are updated as a function of their respective comparison results. In particular, the individual encoder weights of the individual encoder 8a and the individual decoder weights of the individual decoder are updated through back-propagation based on the comparison result obtained by comparing the input data 12 of the modality 2a and the individual reconstructed data 17a. The same is performed for each individual variational autoencoder 10.

As shown in Fig. 5, the steps of training the individual variational autoencoder 10 (steps S6 to S10) are repeated in a step S21 to reduce the comparison results and hence improve the individual variational autoencoders 10. The steps S6 to S10 can be repeated until a desired comparison result is reached or until a predetermined number or runs was performed.

In a step S11 of Fig. 5, the individual latent data of the trained variational autoencoders 10 is used as the multimodal input data 2 of the multimodal variational autoencoder 3 described in view of Fig. 1 to 3. Following step S11, the method of Fig. 5 performs the method steps S1 - S4 with the individual latent data of the trained variational autoencoders 10 being used as the multimodal input data 2 of the multimodal variational autoencoder 3.

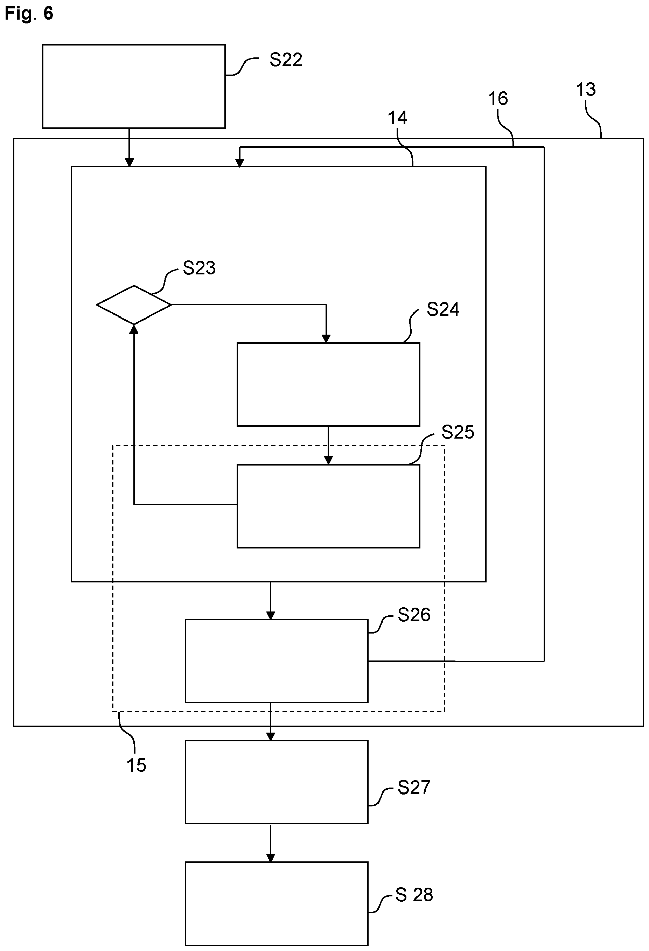

Fig. 6 shows another representation of the training procedure of the neural network 1. In Fig. 6, the boxes 13, 14 and 15 respectively represent a model selection 13, an individual optimization 14 and a hyperparameter optimization 15.

In a step S22, the individual input data 12 of the modalities 2a - 2g is collected. The steps S23 - S25 are part of the individual optimization and include the steps S6 to S11 described in view of Fig. 5. In step S24, the search space of hyperparameters of one individual variational autoencoder 10 is defined (this includes the weights, the number of layers, the activation function, the size of the channel, and the like). In step S25, the architecture of the individual variational autoencoder 10 is optimized, in particular in line with steps S6 to S11. Step S23 indicates that steps S24 and S25 are performed for each modality 2a - 2g. The result of the steps S23 - S25, i.e. the output of the individual optimization 14, is the optimized variational autoencoder 10 for each modality 2a - 2g.

This output is used as an input to step S26, in which the multimodal variational autoencoder 3 is trained for the fixed model architecture defined in steps S23 - S25. Step S26 can include steps S1 - S4 previously defined. Step S26 can include the optimization of the hyperparameters of the latent space. As a result, a joint representation of all modalities 2a - 2g in a latent space 6 is obtained. The optimization in steps S25 and S26 is a hyperparameter Bayesian optimization.

The arrow 16 indicates that the steps S23 - S26 are repeated for different values of the predetermined dimensionality, in order to optimize the loss function of the multimodal variational autoencoder 3 and achieve the best latent space representation of chemical substances.

The hyperparameters for which the loss function is minimized are saved in a step S27. In particular, all information relating to the trained and optimized neural network are stored. This includes latent space variables for each data set together with information on the modalities 2a - 2g and all further available information. In a step S28 of Fig. 6, an application test is run using the trained neural network 1.

Through the training methods described in view of Fig. 1 - 5, a neural network 1 capable of representing a polymer in a latent space representation is provided. In detail, the training data and further data representing polymers can be input into the trained neural network. The trained neural network generates a latent representation of the input data, which can be stored in a database. This allows multiple applications, which will be described in detail below.

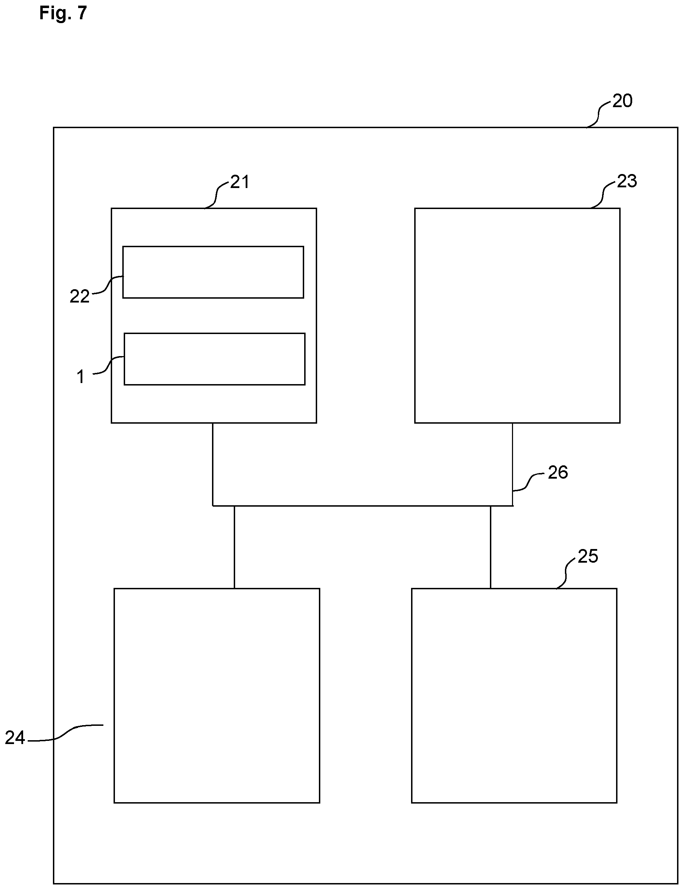

One example of an application of the trained neural network 1 is a database search device 20 (search engine). An example for such a database search device 20 is shown in Fig. 7.

The search device may implement various functions and support a variety of methods, e.g. for generating control data indicative of a synthesis specification for a desired chemical substance, or synthetic measurement data.

The database search device 20 of Fig. 7 includes a storage unit 21, which is a random-access memory (RAM), an input unit 23, a processor 24, which is a CPU, an output unit 25 and a connection cable 26 connecting the different components of the database search device 20.

The database search device 20 is part of a personal computer (PC). The storage unit 21 has a database 22 and the trained neural network 1 stored thereon. The database 22 includes latent space representations of multiple chemical substances (such as polymers) which are obtained from the trained neural network 1. In detail, to obtain the latent space representation stored in the database 22, the trained neural network 1 receives the individual and/or multimodal input data 2, 12 previously used as training data and generates the latent space representation in the latent space 6 using the multimodal and/or individual variational autoencoders 3, 10.

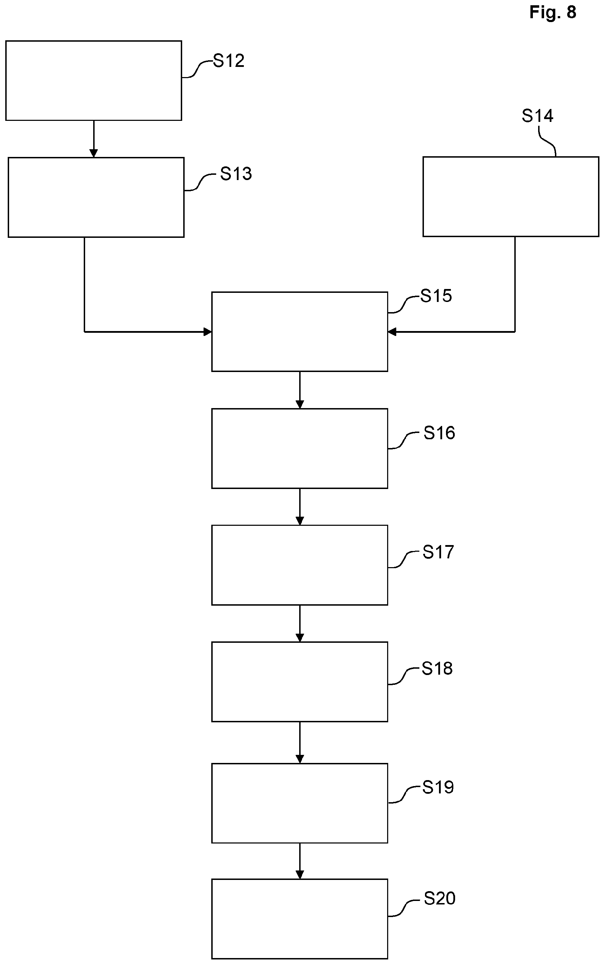

Fig. 8 shows an example of how to use the database search device 20 and Fig. 7 and 8 will be described jointly in the following. The database search device 20 is used to search the database 22 for the same or similar polymers as searched polymer.

In a step S12 of the method of Fig. 8, the input unit 23 receives search input data providing a multimodal representation of a polymer to be searched. The search input data is provided in a multimodal initial space. The search input data has the same format as the previously described multimodal input data 2, with data describing multiple modalities 2a - 2g of the polymer. Optionally, the search input data only includes data describing some of the modalities 2a - 2g.

For example, the search input data include a physicochemical property of a desirable chemical substance, e.g. a specific thermal conductivity. As a result, the method implemented with the database search device 20 outputs control data indicative of a synthesis specification. The control data is suitable to specify the required elements of a chemical plant and to control those to produce the chemical substance which is a polymer in the described example. The control data may include a digital version of a recipe for producing a chemical substance having the desired properties.

In a step S13, the processor 24 is used to transform the search input data into a latent space representation thereof. In detail, the multimodal encoder 4 of the neural network 1 is used to adjust a dimensionality of the search input data to obtain multimodal latent search data in the latent space 6. Thus, a digital representation of the chemical substance, e.g. a polymer, is obtained by deploying the data driven compression model implemented by the encoder 4 and decoder 5.

In a step S14, the processor retrieves the latent space representation of previously known polymers from the database 22 stored in the storage unit 21. The database 22 may include latent space representations of historical or known polymers.

In a step S15 of Fig. 8, the processor 24 compares the latent search data from step S13 with the representations of the multiple polymer retrieved from the database 22. This may involve calculating a similarity score.

In step S16 of Fig. 8, the processor 24 selects at least one polymer from the multiple polymers represented in the database 22 based on a result of the comparison of step S15. The scores may be ranked in step S17 so that a list of similar or close polymers is available in the latent space for further selection.

Here, the processor 24 selects the closest polymer(s) in the latent space 6 (for example, the closest Euclidian distance between points representing the polymers in the latent space 6).

In an optional step S17, the selected closest polymers are ranked by distance, i.e. in accordance with their similarity to the latent search data.

In a step S18 of Fig. 8, an identifier including information about analytical data, a polymer name, a synthesis specification and the like which relate to the selected polymer(s), is retrieved from the database 22.

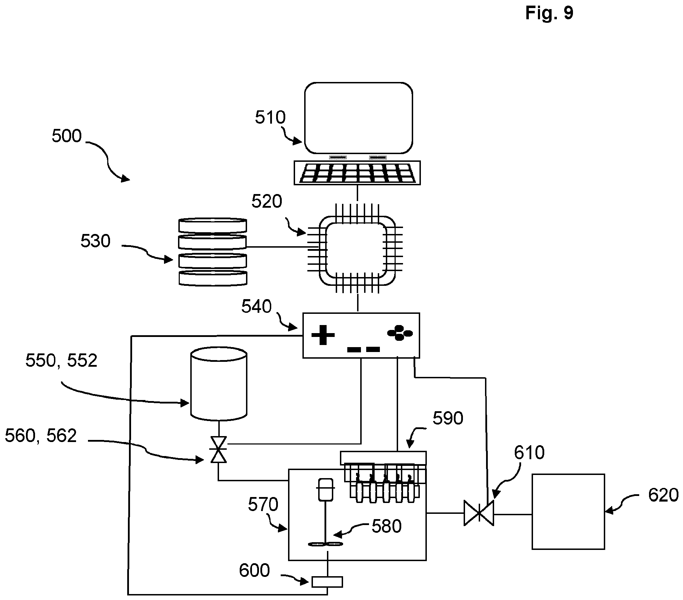

In a step S19, the output unit 25, which is a display, outputs the identifier of the selected polymer(s). The identifier is also stored in the database 22. The output identifier and/or its associated synthesis specification is used to control synthesis of the new (searched) polymer in step S20. The identifier allows for retrieving a prescribed synthesis specification associated to the identified polymer from a specification database 530 (see Fig. 9). Step S20 may involve running an application test.

Fig. 9 shows a system 500 for producing a chemical substance based on a synthesis specification generated according to the above aspects and embodiments of methods and apparatuses generating control data. In this example the system comprises a user interface 510 and a processor 520, associated with a control unit 540, the control unit 540 is configured to receive control data generated according to this disclosure. In this example the control data is provided from a data base 530, in other examples, the control data may be provided from a server. For example, the identifier for a specific set of control data is obtained according to step S18, wherein the identifier refers to its associated synthesis specification and respective control data set.

Vessels 550, 552 each contain a component of the chemical product. In general, more than two vessels may be present. For illustration purposes the example only shows two vessels. Valves 560, 562 are associated with vessels 550, 552. Valves 550 and 552 may be controlled to dose appropriate amounts of each component as an ingredient for synthesizing the selected polymer (step S17) in reactor 570, according to the synthesis specification. A motor 600 of a mixer 580 may also be controlled by the control unit 540 as a function of the control data/synthesis specification. An optional heater 590 may also be controlled according to the synthesis specification. Finally, an exit valve 610 in fluid communication with the reactor may be controlled by the control unit to provide the chemical product to a container or test system 620.

Although the present invention has been described in accordance with preferred embodiments, it is obvious for the person skilled in the art that modifications are possible in all embodiments. For example, the modalities 2a - 2g may be other modalities than the ones described above. The neural network 1 can used for other applications than the database search device 20 described above. Such applications for example include polymer re-engineering based on the output identifier, polymer synthesis based on the output identifier, new polymer design based on the output identifier, reduction of shot learning and the like.

In alternative embodiments and applications of the trained autoencoder or database search device, synthetic measurement data for a chemical substance is obtained based on available sensor data and the underlying data driven compression model. One may also contemplate of generating data indicative of a second physicochemical property based on a second physicochemical property, wherein the first and second properties relate to different modalities in terms of the multimodal latent space representation.

It is understood that all disclosed methods may be implemented as computer-implemented methods. In all methods involving generating control data, the optional step of producing a chemical substance according to the synthesis specification and/or the control data may be carried out by employing a system for producing a chemical substance having a control unit.

### REFERENCE NUMERALS

- 1: neural network
- 2: multimodal input data
- 2a-2g: modality
- 3: multimodal variational autoencoder
- 4: multimodal encoder
- 5: multimodal decoder
- 6: latent space
- 7: multimodal reconstructed data
- 7a - 7g: multimodal reconstructed data
- 8: individual encoder
- 8a - 8g: individual encoder
- 9: individual decoder
- 9a - 9g: individual decoder
- 10: individual variational autoencoder
- 12: individual input data
- 13: model selection
- 14: individual optimization
- 15: hyperparameter optimization
- 16: arrow
- 17: individual reconstructed data
- 17a - 17g: individual reconstructed data
- 20: database search device
- 21: storage unit
- 22: database
- 23: input unit
- 24: processor
- 25: output unit
- 26: connection cable
- 500: system for producing chemical substance/chemical plant
- 510: interface
- 520: processor
- 530: data base
- 540: control unit
- 550, 552: vessel
- 560, 562: valve
- 570: component/ingredient
- 580: mixer
- 590: heater
- 600: motor
- 610: exit valve
- S1: receiving multimodal input data
- S2: adjusting/reducing dimensionality of multimodal input data by encoding based on data driven compression model
- S3: decoding based on data driven compression model
- S4: calculating loss function
- S5: updating en-/decoder weights
- S6: repeating step S1 - S5
- S7: adjusting dimensionality
- S8: decoding individual latent data based on data driven compression model
- S9: comparing individual latent data with reconstructed data
- S10: updating en-/decoder weights
- S11: use individual latent data as multimodal input data
- S12: receiving multimodal representation/measured data
- S13: encoding search input data/transformation into latent space
- S14: retrieving latent space representation from known/historic chemical substances
- S15: comparing search data with known data in latent space/determining distances in latent space
- S16: determining closest points between search and known chemical substances in latent space/selecting substance
- S17: ranking a set of chemical substances closest to the latent space representation of the search input data according to latent space distance/selecting substance
- S18: retrieving identifier for selected/closest chemical substance
- S19: retrieving/generating control data indicative of a synthesis specification for the closest chemical substance
- S20: controlling synthesis of selected chemical substance in accordance with synthesis Specification/running test application
- S21: repeating steps S6 - S10
- S22: receiving multimodal input data
- S23: executing steps for each modality
- S24: setting search space of individual autoencoder
- S25: optimizing architecture of variational autoencoder
- S26: training variational autoencoder
- S27: storing hyperparameters
- S28: running application test

## Claims

1. A method for generating a compressed digital representation of a chemical substance, in particular polymers, in, the method including:
receiving (S12) input data being a multimodal representation (2, 12) of a physicochemical property of the chemical substance and indicative of a measurable physicochemical property of the chemical substance;
encoding (S13) said input data (2, 12) using a data driven compression model (3, 10) of the chemical substance for generating encoded substance data as a function of the received input data (2, 12); and
generating chemical substance data indicative of the measurable physicochemical property of the chemical substance by decoding said encoded substance data using the data driven compression model (3, 10).

2. The method of claim 1, further comprising:
providing (S1), as an input to a neural network (1) to be trained, multimodal input data (2) providing a multimodal representation of the chemical substance in a multimodal initial space, the neural network (1) comprising a multimodal variational autoencoder (3) including a multimodal encoder (4) and a multimodal decoder (5), wherein the multimodal encoder (4) includes multimodal encoder layers having a multimodal encoder weight defining how the multimodal encoder layers transform data, and wherein the multimodal decoder (5) includes multimodal decoder layers having a multimodal decoder weight defining how the multimodal decoder layers transform data;
using the multimodal encoder layers, adjusting (S2) a dimensionality of the multimodal input data (2) to obtain multimodal latent data in a latent space (6);
using the multimodal decoder layers, decoding (S3) the multimodal latent data to obtain multimodal reconstructed data in the multimodal initial space;
calculating (S4) a loss function for the multimodal variational autoencoder (3) for a current set of multimodal encoder and decoder weights based on the multimodal input data (2) and the multimodal reconstructed data; and
outputting configuration data indicative of the trained neural network (1) and/or a latent space representation of the chemical substance as a digital representation of the chemical substance; and
providing the trained neural network as the data driven compression model.

3. The method according to claim 2, further including:
updating (S5) the multimodal encoder weight and/or the multimodal decoder weight based on the loss function; and/or
repeating (S6) the steps of providing (S1) multimodal input data (2), adjusting (S2) the dimensionality, decoding (S3) the multimodal latent data, calculating (S4) a loss function, and/or updating (S5) the weights to reduce the loss function.

4. The method of any one of claims 2 or 3, wherein the neural network (1) further includes an individual variational autoencoder (10) assigned to each modality (2a - 2g), the individual variational autoencoder (10) including an individual encoder (8) and an individual decoder (9), wherein the individual encoder (8) includes individual encoder layers having an individual encoder weight defining how the individual encoder layers transform data, and wherein the individual decoder (9) includes individual decoder layers having an individual decoder weight defining how the individual decoder layers transform data, the method further comprising:
training each individual variational autoencoder (10) by:
inputting (S6) into the individual variational autoencoder (10), individual input data (12) representative only of the assigned modality in an individual initial space;
using the individual encoder layer, adjusting (S7) a dimensionality of the individual input data (12) to obtain individual latent data with a predetermined dimensionality;
using the individual decoder layer, decoding (S8) the individual latent data to obtain individual reconstructed data (17) in the individual initial space;
comparing (S9) the individual input data (12) with the individual reconstructed data (17) to obtain a comparison result;
updating (S10) the individual encoder weight and/or the individual decoder weight based on the comparison result; and
repeating (S21) the steps of inputting (S6) individual input data (12), adjusting (S7) a dimensionality of the individual input data (12), decoding (S8) the individual latent data, comparing (S9) the data and updating (S10) the individual encoder weight and/or the individual decoder weight to reduce the comparison result;
and the method further comprising
using (S11) the individual latent data from the multiple individual variational autoencoders (10) as the multimodal input data (2) of the multimodal variational autoencoder (3).

5. The method of claim 4, further including:
repeating the training of each individual variational autoencoder (10) for a different predetermined dimensionality of the individual latent data; and
selecting the predetermined dimensionality for which the loss function is smallest as the predetermined dimensionality of the final trained neural network (1).

6. The method according to any one of claims 1-5, wherein the loss function for the multimodal variational autoencoder (3) is determined through a mixture of expert or through a product of expert technique.

7. The method according to any one of claims 1-6, wherein one of the modalities (2a - 2g) describing the chemical substance provides: a spectroscopic representation, a rheologic representation, a thermal representation, a chemical representation, a structural representation, a representation of the solubility, a representation of the dispersion, a representation of the viscosity and/or a representation of the surface tension of the chemical substance.

8. A method for measuring a physicochemical property of a chemical substance, comprising:
Receiving (S12) sensor data (2, 12) indicative of a first measurable physicochemical property of the chemical substance;
Encoding (S13) said sensor data (2, 12) using a data driven compression model (3, 10) of the chemical substance for generating encoded sensor data; and
generating measurement data indicative of a second measurable physicochemical property of the chemical substance by decoding said encoded sensor data using the data driven compression model (3, 10);
wherein the data driven compression model (3, 10) is implemented to map input data (2, 12) to encoded output data (6), in particular according to the method of any one of claims 1-7, said input data being a multimodal representation (2, 12) of a physicochemical property of the chemical substance, and said encoded output data being a latent space representation (6) of the input data (2, 12).

9. A measurement apparatus for measuring a physicochemical property of a chemical substance, comprising:
an interface device implemented to receive sensor data (2, 12) indicative of a first measurable physicochemical property of the chemical substance;
an encoder device (4) implemented to encode received sensor data (2, 12) and to generate and output encoded sensor data (6); and
a decoder device (5) implemented to generate measurement data (7, 17) indicative of a second measurable physicochemical property of the chemical substance, and to decode said encoded sensor data (6);
wherein the encoder device (4) is implemented to map input data (2, 12) to encoded output data (6), in particular according to the method of any one of claims 1-7, said input data (2, 12) being a multimodal representation of a physicochemical property of the chemical substance in the multimodal initial space, and said encoded output data (6) being a latent space representation of the input data (2, 12); and
wherein the decoder device (5) is implemented to map input data (6) to decoded output data (7, 17), in particular according to the method of any one of claims 1-7, said input data (6) being a multimodal latent space representation of a physicochemical property of the chemical substance, and said decoded output data (7, 17) being multimodal reconstructed data in the multimodal initial space.

10. The method or apparatus of claim 9 or 10, wherein at least one of the group of: the generated measurement data, recipe data indicative of the chemical substance, identification data indicative of the chemical substance, is output.

11. A database search device (20) for identifying a chemical substance having a predetermined physicochemical property comprising:
a storage unit (21) for storing a database (22) and a trained neural network (1), the database (22) comprising representations of multiple chemical substances having physicochemical properties obtained using the trained neural network (1), and the trained neural network (1) comprising anencoder (3) and a decoder (5);
an input unit (23) for receiving search input data providing a representation indicative of the predetermined physicochemical property of the chemical substance to be searched
a processor (24) configured to:
use the encoder (4) to adjust a dimensionality of the search input data to obtain encoded search data;
compare the encoded search data with the representations of the multiple chemical substances in the database (22); and
select at least one chemical substance from the multiple chemical substances represented in the database (22) based on a result of the comparison between the encoded search data and the representations of the multiple chemical substances; and
an output unit (25) for outputting an identifier indicative of the selected chemical substance and/or a synthesis specification associated to the identifier for the selected chemical substance.

12. The database search device of claim 11, wherein the neural network (1) is trained according to the method of any one of claims 2-7.

13. A method for generating control data indicative of a synthesis specification for a chemical substance, in particular a polymer, comprising:
providing a first synthesis specification for a reference chemical substance;
encoding (S13) the first synthesis specification using a data driven compression model (3, 10) into a digital representation (6) of the reference chemical substance;
providing a database (22) comprising a plurality of historical digital representations of historical chemical substances;
determining a similarity score (S16) for the historical digital representations with respect to the digital representation of the reference chemical substance;
based on the similarity score, selecting (S17) at least one historical representation, and decoding generating a synthesis specification associated with the least one selected historical representation; and
generating control data indicative of the generated synthesis specification.

14. The method of claim 13, wherein the data driven compression model (3, 10) is implemented to map input data (2, 12) to encoded output data (6) according to the method of any one of claims 1-7, said input data being a multimodal representation (2, 12) of a chemical substance, and said encoded output data being a latent space representation (6) of the input data (2, 12).

15. A method for generating control data indicative of a synthesis specification for a chemical substance, in particular a polymer, comprising:
receiving (S12) sensor data (2, 12) indicative of a measurable physicochemical property of the chemical substance;
encoding (S13) said sensor data (2, 12) using a data driven compression model (3, 10) of the chemical substance for generating encoded sensor data; and
generating control data indicative of a synthesis specification for the chemical substance by decoding said encoded sensor data using the data driven compression model (3, 10);
wherein the data driven compression model (3, 10) is implemented to map input data (2, 12) to encoded output data (6) according to the method of any one of claims 1-7, said input data being a multimodal representation (2, 12) of a physicochemical property of the chemical substance, and said encoded output data being a representation (6) of the input data (2, 12).
